# EUROPEAN PATENT APPLICATION

(11) **EP 4 403 568 A1**
(43) Date of publication of application: **24.07.2024**
(21) Application number: 21957117.1
(22) Date of filing: 17.09.2021
(51) Int. Cl.: C07K 14/78, C07K 1/36, A61K 38/39, A61L 31/00, A61L 27/24, C08L 89/06

(54) **USE OF COLLAGEN PARTICLES TO PROMOTE HAIR FOLLICLE FORMATION OR ANGIOGENESIS**

(71) Applicant: Acro Biomedical Company. Ltd., Kaohsiung City 82151 (TW)
(72) Inventor: CHEN, Yun-Ju, Taiwan 82151 (TW); WEI, Chao-Yi, Taiwan 82151 (TW); HSIEH, Dar-Jen, Taiwan 82151 (TW)
(74) Representative: Lang, Christian
(86) International application number: PCT/CN2021/119067
(87) International publication number: WO 2023/039833

(57) **Abstract**

Disclosed herein is use of collagen particles for preparation of a medicament for inducing hair follicles neogenesis or angiogenesis in a subject. The collagen particles in the present application have a diameter of about 10-200 µm. According to some embodiments of the present disclosure, the collagen particles are administered to the subject in an amount of about 0.1 mg/cm² to about 1,000 mg/cm²

## Description

### BACKGROUND OF THE INVENTION

### FIELD OF THE INVENTION

The present disclosure relates to the field of medication. More particularly, the present disclosure relates to methods for inducing hair follicle neogenesis or angiogenesis by using collagen particles.

### 2. DESCRIPTION OF RELATED ART

Skin is the largest organ in human body that modulates body temperature and moisture, and forms the first line of defense against pathogens. There are several skin appendages (e.g., hair follicles, sweat glands, etc.) present in the dermis, and the damage of the dermis often leads to severe consequences.

Specifically, hair grows from the hair follicles of the dermis, and the hair follicles is composed of various types of cells, including hair follicle stem cells. The life cycle of the hair follicles is divided into three stages, including anagen stage, catagen stage, and telogen stage. The hair (*i.e.* the hair shaft with the cuticle structure) forms in the anagen stage, in which the hair follicle stem cells proliferate and differentiate into keratinocyte stem cells, and the keratinocyte stem cells further differentiate into matrix keratinocytes. The derivatives of the matrix keratinocytes form the outer root sheath and inner root sheath of the hair follicle. The activated hair follicle stem cells may form epidermal stem cells that differentiate into epidermal cells. Furthermore, the activated hair follicle stem cells may differentiate into sebaceous glands. In the catagen and telogen stages, hair no longer grows from the hair follicles, and the hair follicles start shrinking. Accordingly, severe dermis injuries caused by aberrant hair follicle cycle or hair follicle damage would lead to irreversible hair loss or baldness.

In addition, the skin injuries caused by burn, ulcer, inflammation, radical therapy, surgery, and diabetes result in vascular atrophy and necrosis leading to chronic wound in subjects. In clinical practice, chronic wound exhibits continuous inflammation and impaired angiogenesis. Thus, the injury and aberrance of skin not only cause infection, but also affect the normal function of skin appendages (*e.g.,* hair loss or insufficient blood supply in local tissue) that significantly impacts physical and mental health. However, none of the drugs used currently in clinics can solve the problems.

In view of the forging, there exists in the related art a need for a medicament or a method that can effectively induce hair follicle neogenesis or angiogenesis.

### SUMMARY

The following presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later.

The present disclosure is aimed to provide a method of inducing hair follicle neogenesis and/or angiogenesis. As embodied and wildly descripted in the present disclosure, the first aspect of the present disclosure is directed to the use of a collagen particle for the preparation of a medicament, in which the medicament is useful for inducing hair follicle neogenesis and/or angiogenesis in a subject, and the medicament comprises a plurality of the collagen particle.

According to some embodiments of the present disclosure, the collagen particle is about 10-200 µm in diameter. Preferably, the collagen particle is about 100-150 µm in diameter.

According to certain embodiments of the present disclosure, the medicament comprising a plurality of the collagen particle is parenterally administered to a desired target site. Examples of parenteral routes include, but are not limited to, percutaneous, intradermal, and subcutaneous administration. The collagen particles are present in the medicament in the amount of 0.1 to 1,000 mg/cm². Preferably, the collagen particles are present in the medicament in the amount of 5 mg/cm².

According to some embodiments of the present disclosure, the medicament comprising a plurality of collagen particles is subcutaneously administered to the subject.

In some embodiments of the present disclosure, the collagen particle is prepared by a method comprising steps of,
(1) subjecting an animal skin about 0.1-2 mm in thickness to a first flow of supercritical carbon dioxide (scCO₂) at a pressure of about 100-500 bar and a temperature of about 30-50°C for a period of about 20 minutes to 10 hours to decellularize the animal skin;
(2) subjecting the decellularized animal skin of step (1) to an alkaline solution;
(3) subjecting the alkaline solution treated animal skin of step (2) to a hydrogen peroxide solution;
(4) subjecting the hydrogen peroxide solution treated animal skin of step (3) to a second flow of scCO₂ in the presence of a co-solvent (or auxiliary solvent) under a pressure of about 100-500 bar and a temperature of about 30-50°C for about 20 minutes to 10 hours to produce a collagen scaffold; and
(5) dehydrating and granulating the collagen scaffold of step (4) to produce the collagen particle.

According to embodiments of the present disclosure, the present method is characterized in not using any organic solvent or cross-linking agent.

According to some embodiments of the present disclosure, in step (1), the animal skin is subjected to the first flow of scCO₂ at the pressure of 350 bar and the temperature of 40°C for 90 minutes.

According to one preferred embodiment, the alkaline solution of step (2) is a solution of sodium hydroxide (NaOH).

According to one preferred embodiment of the present disclosure, in step (4), the animal skin is subjected to the second flow of scCO₂ at the pressure of 350 bar and the temperature of 40°C for 90 minutes.

In a preferred embodiment of the present disclosure, the co-solvent of step (4) is ethanol. In one example, the co-solvent and the second flow of scCO₂ are present in a volume ratio of 1:10.

In another embodiment of the present disclosure, in step (5), the collagen scaffold is cut or grounded into a plurality of the collagen particle in liquid nitrogen.

Another aspect of the present disclosure is directed to a method of inducing hair follicle neogenesis and angiogenesis in a subject. The method comprises administering to the subject an effective amount of a medicament comprising said plurality of collagen particle of the present disclosure, wherein the collagen particle has a diameter of 10-200 µm.

According to some embodiments of the present disclosure, each of the collagen particle is about 100-150 µm in diameter.

According to certain embodiments, the medicament comprising a plurality of the collagen particle is administered in the amount of about 0.1-1,000 mg/cm². Preferably, the medicament comprising a plurality of the collagen particle is administered in the amount of about 5 mg/cm².

In some embodiments of the present disclosure, the medicament comprising a plurality of the collagen particle is subcutaneously administered to the subject.

According to some embodiments of the present disclosure, the collagen particle is prepared by a method comprising:
(1) subjecting an animal skin of 0.1-2 mm in thickness to a first flow of supercritical carbon dioxide at a pressure of about 100-500 bar and a temperature of about 30-50°C for about 20 minutes to 10 hours so as to decellularize the animal skin;
(2) subjecting the decellularized animal skin of step (1) to an alkaline solution;
(3) subjecting the alkaline solution treated animal skin of step (2) to a hydrogen peroxide solution;
(4) subjecting the hydrogen peroxide solution treated animal skin of step (3) to a second flow of supercritical carbon dioxide in the presence of a co-solvent at a pressure of about 100-500 bar and a temperature of about 30-50°C for about 20 minutes to 10 hours to produce a collagen scaffold; and
(5) dehydrating and granulating the collagen scaffold of step (4) to produce the collagen particle.

In one embodiment of the present disclosure, the method does not use any organic solvent or cross-linking agent.

According to some embodiments, in step (1), the animal skin is subjected to the first scCO₂ under a pressure of 350 bar and a temperature of 40°C for 90 minutes.

In one preferred embodiment of the present disclosure, the alkaline solution is NaOH.

According to one preferred embodiment, in step (4), the animal skin is subjected to the second scCO₂ under a pressure of 350 bar and a temperature of 40°C for 90 minutes.

In some preferred embodiments of the present disclosure, in step (4), the co-solvent is ethanol. In one example, the co-solvent and the scCO₂ are present in a volume ratio of 1:10.

According to another embodiment, in step (5), the collagen scaffold is cut or ground into the collagen particle in liquid nitrogen.

Based on the above, the present method is useful for inducing hair follicle neogenesis and angiogenesis in a subject via administrating to the subject an effective amount of a medicament containing the collagen particle so as to treat hair follicle loss and/or vascular injury in the subject.

Many of the attendant features and advantages of the present disclosure will becomes better understood with reference to the following detailed description considered in connection with the accompanying drawings.

### BRIEF DESCRIPTION OF THE DRAWINGS

The present description will be better understood from the following detailed description read in light of the accompanying drawings, where:

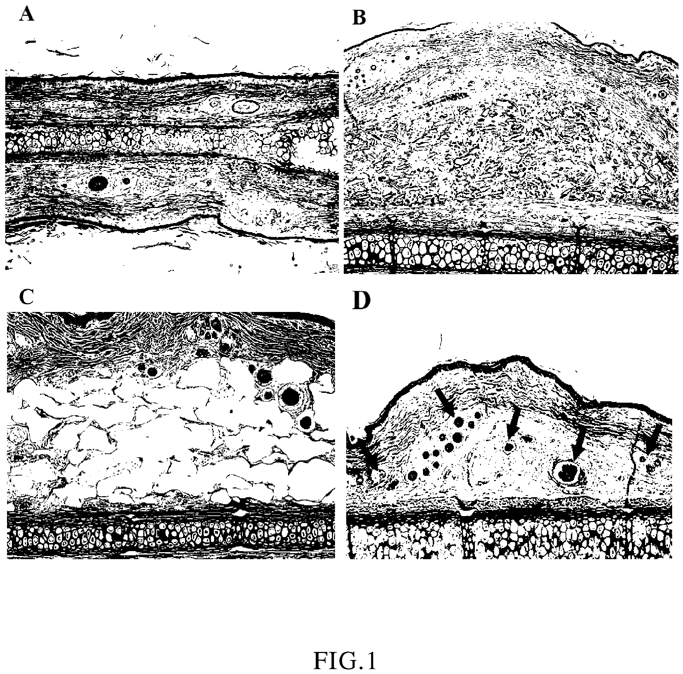

FIG. 1 is directed to the photographs of tissue staining according to one example of the present disclosure, in which (A) normal saline (control 1), (B) poly-L-lactic acid (control 2), (C) hyaluronic acid (control 3), and (D) the present collagen particle were subcutaneously injected to the ears of rabbits, respectively; 30 days later, the tissues were isolated from the ears, followed by staining and detected by microscope; the photographs were taken under an amplification factor of 40x; black arrow: vascular tissue; red arrow: hair follicle cell.

### DESCRIPTION

The detailed description provided below in connection with the appended drawings is intended as a description of the present examples and is not intended to represent the only forms in which the present example may be constructed or utilized. The description sets forth the functions of the example and the sequence of steps for constructing and operating the example. However, the same or equivalent functions and sequences may be accomplished by different examples.

### I. Definition

For convenience, certain terms employed in the specification, examples and appended claims are collected here. Unless defined otherwise, all technical and scientific terms used herein have the same meaning as commonly understood by one of the ordinary skill in the art to which this invention belongs. The singular forms "a", "and", and "the" are used herein to include plural referents unless the context clearly dictates otherwise.

The term "collagen particle" refers to a granulated collagen prepared by a method disclosed in the present disclosure, in which the collagen particle is produced by decellularizing, sterilization, and grinding from an animal source to a specific size. Specifically, the animal skin is decellularized by scCO₂ to produce a collagen scaffold without using any crosslinking agent, the collagen scaffold is then grounded to produce the collagen particle having a specific diameter while retaining the structure and configuration of a nature collagen.

The term "administering", "administered" and "administration" refer to a mode of providing the collagen particle to a subject to alleviate and ameliorate the conditions related to hair follicle defection and angiogenesis.

The term "effective amount" refers to the amount of the collagen particle or a medicament comprising the collagen particle administered in a period of time to yield a desired effect on a disease, or to delay or minimize symptoms related to the disease, so as to induce hair follicle neogenesis and angiogenesis. The effective amount of the collagen particle or the medicament of the present disclosure refers to an amount that can show beneficial effects against diseases when administered alone or combined with other therapeutic agents. According to one example of the present disclosure, the amount of the collagen particle or the medicament can induce hair follicle neogenesis and angiogenesis in the dermis of a subject.

The term "subject" refers to a mammal including the human species treatable with the method of the present invention. The mammal refers to all members of Mammalia, including human, primates, domestic animals, livestock, captive animals, sports animals, and pets; and rodents. In addition, the term "subject" or "patient" is intended to refer to both the male and female unless one gender is specifically indicated.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about". Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

### II. Detail description of the invention

The present disclosure is based, at least in part, on the discovery that the collagen particle, which is prepared by decellularizing and grinding without using any crosslinking agent during the procedure, may serve as a three-dimension biological scaffold for cells to grow thereon *in vivo.* Furthermore, the collagen particles may also induce the hair follicle neogenesis and angiogenesis. Accordingly, the present disclosure aims at providing a medicament comprising the present collagen particles, in which the medicament is useful for repairing skin injuries such as by inducing hair follicle neogenesis or angiogenesis in a subject.

One aspect of the present disclosure is directed to a method of treating hair follicle defection or local vascular injury in a subject. The method comprises administrating to the subject an effective amount of the collagen particle; preferably, the collagen particle is subcutaneously administered to the subject to induce local hair follicle neogenesis and angiogenesis.

The collagen particle may be prepared from animal tissues according to methods known to skilled artisans or methods of the present disclosure, with or without using crosslinking agents in the preparation process. In one example of the present disclosure, the animal skin is decellularized via scCO₂ and treated with agents including alkaline solution and hydrogen peroxide, followed by grinding to produce collagen particles with specific sizes. Note that no organic solvents (especially those that are known to be toxic to organisms) and crosslinking agents are used in the process. The collagen particle thus produced may be stored *in vitro* via any methods known in the field, for example, by storing in a solution. The collagen particles produced by the method of present disclosure independently retains its natural structure, signal factor, and configuration of a nature collagen, thus may serve as a better microenvironment for cell and tissue to grow thereon.

The collagen particles suitable for use in the present disclosure may be isolated from allogeneic and/or xenogeneic tissues. Allogeneic collagen particles refer to those obtained from tissues or cells of the same species but different individuals; xenogeneic collagen particles refer to those obtained from individuals of different species. Specifically, the collagen particles may be isolated from skin tissues, tendon tissues, or any tissue rich in collagen, these tissues may be from individuals of the same species or different species. Examples of the sources of tissues in the present disclosure include, but are not limited to, skin tissues, tendon tissues, and cartilage tissues. According to certain embodiments, the collagen particles are derived from pig skin tissues.

Specifically, the collagen particle of the present disclosure is prepared by the method comprising following steps without using any organic solvents or cross-linking agents,
(1) subjecting an animal skin about 0.1-2 mm in thickness to a first flow of scCO₂ at a pressure of about 100-500 bars and a temperature of about 30-50°C for about 20 minutes to 10 hours so as to decellularize the animal skin;
(2) subjecting the decellularized animal skin of step (1) to an alkaline solution;
(3) subjecting the alkaline solution treated animal skin of step (2) to a hydrogen peroxide solution;
(4) subjecting the hydrogen peroxide solution treated animal skin of step (3) to a second flow of scCO₂ in the presence of a co-solvent at a pressure of about 100-500 bar and a temperature of about 30-50°C for about 20 minutes to 10 hours to produce a collagen scaffold; and
(5) dehydrating and granulating the collagen scaffold of step (4) to produce the collagen particle.

Prior to commencing the present method, the skin tissue of an animal is preferably subjected to washing, depilation, and lipid extraction processes. Animals that may serve as the source of a skin tissue for use in the present invention are economic animals, which include, but are not limited to, pig, cattle, cow, bull, sheep, goat, donkey, rabbit, duck, goose, and chicken. The depilation and lipid extraction may be performed by using any physical or chemical methods known in the art. For example, the skin tissue can be subjected to an acid treatment to remove hairs thereon or can be subjected to enzymatic (e.g., lipase) or chemical (e.g., detergent) treatment to remove lipids therefrom. Alternatively, the lipid can be directly cutting away by use of a knife or cutting means.

Then, the skin tissue described above is preferably sliced into a skin of about 0.1-2 mm in thickness, for example, about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 mm Preferably, the animal skin is about 0.3 mm in thickness.

In step (1), the hairless and fat-free animal skin about 0.1-2 mm in thickness is decellularized via a first flow of scCO₂. The purpose of step (1) is to remove cellular components from the animal skin while preserving the physical and biochemical properties of collagen, so that it can be used as a tissue scaffold. Preferably, in step (1), the animal skin is subjected to the first flow of scCO₂ under a pressure of about 100-500 bars, such as 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490 or 500 bar; more preferably, about 200-400 bar, such as 200, 210, 220, 230, 240, 250, 260, 270, 280, 290, 300, 310, 320, 330, 340, 350, 360, 370, 380, 390 or 400 bar. Furthermore, step (1) is carried out at a temperature about 30-50°C, for example, about 30, 31, 32, 33, 34, 35, 36, 37, 38, 39, 40, 41, 42, 43, 44, 45, 46, 47, 48, 49 and 50°C; preferably, step (1) is carried out at a temperature about 35-42°C. The treatment in step (1) lasts for about 20 minutes to 10 hours (e.g., 20, 30, 40, 50, or 60 minutes, or 2, 3, 4, 5, 6, 7, 8, 9, or 10 hours). According to some embodiments of the present disclosure, the animal skin about 0.1-2 mm in thickness is subjected to scCO₂ at a temperature of 40°C and a pressure of 350 bar for 90 minutes. In essence, the decellularization is carried out at a temperature close to body temperature (i.e., 37°C) to remove biologically active substances.

In step (2), the decellularized animal skin of step (1) is subjected to an alkaline solution to wash and remove any cellular residues. Specifically, the animal skin is soaked in the alkaline solution for 0.5-2 hours, for example, about 0.5, 1, 1.5, or 2 hours; preferably, about 2 hours. Example of the alkaline solution suitable for use in the present disclosure includes, but is not limited to: sodium hydroxide solution, calcium hydroxide solution, potassium hydroxide solution, sodium carbonate solution, sodium bicarbonate solution, etc. According to one preferred embodiment of the present disclosure, the decellularized animal skin of step (1) is treated with a sodium hydroxide solution at a concentration of 1N.

In step (3), the alkaline solution treated animal skin of step (2) is further treated with a hydrogen peroxide solution for about 0.5-2 hours, e.g., 0.5, 1, 1.5, and 2 hours. In some preferred embodiments, the hydrogen peroxide solution has a concentration about 0.1-2% by weight, for example, about 0.1, 0.2, 0.3, 0.4, 0.5, 0.6, 0.7, 0.8, 0.9, 1, 1.5, or 2% by weight. According to one preferred embodiment, the alkaline solution treated animal skin of step (2) is treated with 1% hydrogen peroxide solution for about one hour.

Next, in step (4), the hydrogen peroxide solution treated animal skin produced in step (3) is subjected to a second flow of scCO₂ in the presence of a co-solvent to produce a collagen scaffold essentially devoid of any cells and bioactive components. Note that step (4) is carried out at a condition similar to that of step (1), except in step (4), the animal skin is treated with scCO₂ along with the co-solvent. That is, the animal skin of step (3) is treated with scCO₂ in the presence of a co-solvent at a pressure of 100-500 bar and a temperature of 30-50°C for 20 minutes to 10 hours. According to preferred embodiments of the present disclosure, in step (4), the hydrogen peroxide solution treated animal skin is subjected to scCO₂ in the presence of a co-solvent at a temperature of 40°C and a pressure of 350 bar for 90 minutes.

The co-solvent may be a C₁₋₄ alcohol, example of which includes, but is not limited to, ethanol, propanol, isopropanol, butanol, isobutanol, 2-butanol, 2-methyl-2-propanol, and cyclobutanol. In certain preferred embodiments, the co-solvent is ethanol and is used along with scCO₂, in which the co-solvent and the scCO₂ are present at a volume ratio of 1:20 to 1:4, for example, 1:20, 1:19, 1:18, 1:17, 1:16, 1:15, 1:14, 1:13, 1:12, 1:11, 1:10, 1:9, 1:8, 1:7, 1:6, 1:5, or 1:4. In one preferred embodiment, the ethanol and scCO₂ are present at a volume ratio of 1:10.

Finally, in the granulating step (*i.e.,* step (5)), the collagen scaffold of step (4) is cut or grounded in liquid nitrogen to produce the collagen particle having a specific diameter. According to some embodiments of the present disclosure, the collagen particle is about 10-200 µm in diameter, such as10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 µm in diameter. In some preferred embodiments, the collagen particle is about 100-150 µm in diameter, for example, about 100, 105, 110, 115, 120, 125, 130, 135, 140, 145, or 150 µm in diameter.

The aforementioned method is characterized by using intact animal skin as starting material, followed by decellularization to remove cellular matrix and active components, and then subjecting to a grinding treatment. The whole process does not use any strong acid and/or strong base; thus, it is not necessary to use cross-linking agents to induce the major amino acids of collagen (*i.e.,* glycine, proline etc.) to polymerize into secondary or tertiary structures. Therefore, the thus-produced collagen particle is characterized by having collagen fibers that retain their nature structure and conformation, allowing the collagen particle to use as a biological scaffold for cell growth.

According to some embodiments of the present disclosure, the administration of the collagen particle prepared by the abovementioned method induces hair follicle neogenesis and angiogenesis in a subject. According to certain embodiments of the present disclosure, the collagen particle is administered to the subject in an amount of 0.1-1,000 mg/cm² skin area, such as 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 150, 200, 250, 300, 350, 400, 450, 500, 550, 600, 650, 700, 750, 800, 850, 900, 950, or 1,000 mg/cm² skin area. Preferably, the collagen particle is administered to the subject in an amount of about 0.1-500 mg/cm² skin area, such as 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 150, 200, 250, 300, 350, 400, 450, or 500 mg/cm² skin area. More preferably, the collagen particles is administered in an amount of about 0.1-50 mg/cm² skin area, for example, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 mg/cm² skin area. In one specific embodiment of the present disclosure, the collagen particle is administered to a subject in need thereof in an effective amount of about 5 mg/cm². Additionally, the collagen particle can be administered at a frequency ranging from once every day to once every three months. In some embodiments, the collagen particle of the present disclosure is administered at a frequency of once a day, once every two days, once every three days, once a week, once every two weeks, once a month, once every two months, or once every three months. In certain embodiments, the collagen particle is administered at a frequency of once a week to once a month.

The collagen particle may be administered to a desired target site through a suitable route. In some embodiments, the collagen particle is administered to a desired target site via non-oral or parenteral administration. Exemplary non-oral administration routes include, but are not limited to, transdermal, intradermal, and subcutaneous administration. Specifically, the non-oral administration route refers to subcutaneous administration (*e.g.,* subcutaneous injection) and/or transdermal administration of the collagen particle to the target site (*i.e.,* applying the collagen particle topically to a subject's skin wound using an applicator). It would be appreciated that a skilled artisan may understand that the suitable routs will vary with the disease being treating, the severity of a disease, the health condition of the subject (including age, physiological state, body type, gender and weight), the duration of treatment, co-existing medical conditions (if any), the dosage and nature of the active ingredient, genetic factors, and other similar factors that commonly known and understood by healthcare professionals. These factors are known in the art and a skilled artisan can implement them without dual experiments. In general, the best route of administration depends on various factors, for example, the stability of the medicament in circulatory system and/or the physical state of the subject (such as whether the subject can tolerate subcutaneous injection). According to some embodiments of the present disclosure, the collagen particle is administered to a subject via subcutaneous injection. In some preferred embodiments, the collagen particle is administered to the dermis of a subject.

The subject mentioned in the present disclosure refers to any animals treatable with the present method having benefit to it. Exemplary animals include, but are not limited to, human, rat, mouse, guinea pig, rabbit, monkey, pig, sheep, cow, horse, dog, and cat. In a working example, the subject is a rabbit. In another example, the subject is a human.

The present disclosure aims to repair skin injuries via administering to a subject in need thereof a collagen particle, which is prepared by the method of the present disclosure, and is composed of the collagen derived from decellularized skin; the collagen particle has a diameter of 10-200 µm and retains its natural structure, signal factor, and intact fiber conformation. Therefore, the collagen particle may serve as a 3D bio-scaffold, which, after administering to the subcutaneous tissue of a subject, provides an environment suitable for cell activation and proliferation, allowing cells (*e.g.,* stem cells, fibroblasts, endothelial cells) to grow thereon. The collagen particle thus provides a mean to induce hair follicle neogenesis and angiogenesis in the dermis of a subject so as to repair skin injuries.

The present invention will now be described more specifically with reference to the following embodiments, which are provided for the purpose of demonstration rather than limitation.

### EXAMPLES

### Materials and Methods

### Animals

New Zealand White rabbits (about 5 kilograms each) were used in the study. Each rabbit was housed in a cage separately in an animal facility with ad libitum access to food and water. In the facility, the temperature was maintained at 19-25°C and the humidity was about 50-60%. The body weight, body temperature, and other physiological values were measured daily. Before each experiment, animals were quarantined and acclimated to the experimental environment.

### Example 1: production of collagen particles

The hairless and fat-free pig skin (0.3 mm in thickness) was dehydrated at4°C for 24 hours, then decellularized by subjecting to scCO₂ under a pressure of 350 bar and a temperature of 40°C for 90 minutes to remove cell components.

Then, the decellularized pig skin was treated, in sequence, with NaOH, hydrogen peroxide solution, low temperature dehydration, and second scCO₂. Specifically, the decellularized pig skin was soaked in 1N NaOH solution for two hours, then in 1% hydrogen peroxide solution for one hour. Then, the pig skin was subjected to a second treatment of scCO₂ in the presence of ethanol as a co-solvent, in which the ethanol and scCO₂ were present at a volume ratio of 1:10, at 350 bar and 40°C for 90 minutes to produce a collagen scaffold free of impurities.

The collagen scaffold was dehydrated at 4°C for 8-30 hours, and grounded into collagen particles independently about 100-150 µm in diameter by a grinder (Retsch, ZX200). The thus-produced collagen particles were irradiated by gamma ray (10-50 kGy), and stored in a sterile condition until further use. The collagen particles produced by the above-mentioned method were examined by electron microscope, and EM photos revealed that the fibril structure of each collagen particle remained intact after grinding.

### Example 2: the collagen particles of Example1 induced hair follicle neogenesis or angiogenesis

To investigate whether the collagen particles of Example 1 could serve as a biological scaffold in skin tissue, one specific area (approximately 1 cm × 1 cm) of a rabbit ear was injected with a collagen solution (100 µL, 35 mg/mL) containing the collagen particles of Example 1 (100-150 µm in diameter); while other areas of the same ear were respectively injected with the same volume of polylactic acid (30 mg/mL), hyaluronic acid (20 mg/mL), or normal saline as the control. After 30 days of the injection, the skin tissues were harvested from the ear and examined by immunohistochemistry staining. The results are presented in Table 1 and FIG.1.

**Table 1. Hair follicle neogenesis and angiogenesis in rabbit subcutis after various treatments**

| | control | polylactic acid | hyaluronic acid | collagen particle of the present disclosure |
|---|---|---|---|---|
| Hair follicle neogenesis | -- | + | + | +++ |
| Angiogenesis | -- | + | + | +++ |

As depicted in FIG. 1, compared to the treatments of normal saline (control group, panel (A) of FIG. 1), polylactic acid (panel (B) of FIG. 1), and hyaluronic acid (panel (C) of FIG. 1), hair follicle neogenesis (panel (D) of FIG. 1, red arrow) and angiogenesis (panel (D) of FIG. 1, black arrow)were detected on the rabbit ear injected with the present collagen particle solution. However, no hair follicle neogenesis or angiogenesis were detected in the area injected with polylactic acid or hyaluronic acid.

In conclusion, the present collagen particles with intact fibril structure can be used as a bio-scaffold *in vivo,* as it retained the subcutaneous tissue that supports the growth of stem cells and fibroblasts, allowing the stem cells to differentiate into specific cells, and inducing the fibroblasts to secrete more growth factors and extracellular matrixes, including *de novo* collagen, to stimulate hair follicle growth and induce angiogenesis in fibroblasts via paracrine signaling.

It will be understood that the above description of embodiments is given by way of example only and that various modifications may be made by those with ordinary skill in the art. The above specification, examples, and data provide a complete description of the structure and use of exemplary embodiments of the invention. Although various embodiments of the invention have been described above with a certain degree of particularity, or with reference to one or more individual embodiments, those with ordinary skill in the art could make numerous alterations to the disclosed embodiments without departing from the spirit or scope of this invention.

## Claims

1. Use of a collagen particle for the preparation of a medicament for inducing hair follicle neogenesis or angiogenesis in a subject, wherein the medicament comprises a collagen particle, and the collagen particle is about10-200 µm in diameter.

2. The use of claim 1, wherein the collagen particle is about 100-150 µm in diameter.

3. The use of claim 1, wherein the collagen particle is administered to the subject in an amount of 0.1-1,000 mg/cm².

4. The use of claim 3, wherein the collagen particle is administered to the subject in an amount of 5 mg/cm².

5. The use of claim 1, wherein the collagen particle is subcutaneously administered to the subj ect.

6. The use of claim 1, wherein the collagen particle is prepared by a method comprising:
(1) subjecting an animal skin about 0.1-2 mm in thickness to a first flow of supercritical carbon dioxide at a pressure of about 100-500 bar and a temperature of about 30-50°C for about 20 minutes to 10 hours to decellularize the animal skin;
(2) subjecting the decellularized animal skin of step (1) to an alkaline solution;
(3) subjecting the alkaline solution treated animal skin of step (2) to a hydrogen peroxide solution;
(4) subjecting the hydrogen peroxide solution treated animal skin of step (3) to a second flow of supercritical carbon dioxide in the presence of a co-solvent at a pressure of about 100-500 bar and a temperature of about 30-50°C for about 20 minutes to 10 hours to produce a collagen scaffold; and
(5) dehydrating and granulating the collagen scaffold of step (4) to produce the collagen particle;
wherein the method does not use any organic solvent or cross-linking agent.

7. The use of claim 6, wherein in step (1), the animal skin is subjected to the first flow of supercritical carbon dioxide at the pressure of 350 bar and the temperature of 40°C for 90 minutes.

8. The use of claim 6, wherein in step (2), the alkaline solution is sodium hydroxide solution.

9. The use of claim 6, wherein, in step (4), the animal skin is subjected to the second flow of supercritical carbon dioxide at the pressure of 350 bar and the temperature of 40°C for 90 minutes.

10. The use of claim 6, wherein in step (4), the co-solvent is ethanol.

11. The use of claim 6, wherein in step (5), the collagen scaffold is cut or ground into the collagen particle in liquid nitrogen.
